Europäisches Patentamt

European Patent Office

Office européen des brevets

(11) Publication number: **0 254 203**
**A2**

## (12) EUROPEAN PATENT APPLICATION

(21) Application number: 87110254.7

(22) Date of filing: 16.07.87

(51) Int. Cl.⁴: **G01N 21/25** , G01N 33/487 , G01N 21/78 , A61B 5/14

(30) Priority: 22.07.86 US 888754

(43) Date of publication of application:
27.01.88 Bulletin 88/04

(84) Designated Contracting States:
CH DE FR GB IT LI NL SE

(71) Applicant: **Personal Diagnostics, Inc.**
**628 Route 10**
**Whippany New Jersey 07981(US)**

(72) Inventor: **Meserol, Peter M.**
**12 Stoneybrook Road**
**Montville New Jersey 07045(US)**
Inventor: **Palmieri, Thomas**
**470 Kossuth Street**
**Paramus New Jersey 07652(US)**

(74) Representative: **LOUIS, PÖHLAU, LOHRENTZ &**
**SEGETH**
**Kesslerplatz 1 Postfach 3055**
**D-8500 Nürnberg(DE)**

(54) Optical analyzer.

(57) An optical analyzer for determining an analyte in a fluid of interest such as a body fluid of interest satisfying the foregoing need and embodying the present invention may include a housing; combination optically transparent cuvette and lancet mounted removably in the housing, the cuvette may receive an optically transparent reagent test system for reacting with the body fluid to produce a change in at least one optical transmissive characteristic of the system indicative of the analyte; a cuvette carrier mounted slidably in the housing and for removably receiving the cuvette; a spring actuator mounted in the housing the connected to the cuvette carrier, the spring actuator may be compressed and released to advance the carrier and thereby advance the lancet into engagement with a portion of a body to produce the body fluid, and the spring actuator retract the lancet within the housing after the advancement; depth control apparatus for controlling the depth of penetration of the lancet into the body portion; an electro-optical system mounted in the housing in optical engagement with the cuvette and for passing a light beam through the cuvette and the reagent system and for receiving the light beam modified by the change in optical transmissive characteristic of the system and for transmitting computation signals indicative of the analyte to a computer; a computer mounted in the housing for receiving the computation signals and for comparing the computation signals against predetermined data to produce display signals indicative of the analyte; a display mounted in the housing and for receiving the display signals and for providing a visible display indicative of the analyte; and control switches mounted in the housing and connected to the computer for controlling the operation of the computer.

FIG.2

# OPTICAL ANALYZER

## Background of the Invention

This invention relates generally to a new and improved optical analyzer for determining analyte in a fluid of interest, and more particularly relates to a new and improved miniaturized optical analyzer for determining analyte in a body fluid of interest and which analyzer is of sufficiently small size and weight to permit the optical analyzer to be taken to the source of the analyte body fluid such as a patient in a hospital bed or a home user.

The term "analyte" as known to those skilled in the art is: a term or expression used to identify generally something that is determined by analysis as to its presence, the amount present, etc. For example, the amount of glucose present in blood is determined by diabetics to monitor the amount of insulin to be taken or to determine whether or not additional insulin should be taken; hence, it will be understood that in this context the "analyte" is blood glucose.

As further known to those skilled in the art, numerous optical analyzers are known for determining analytes in many different fluids of interest such as body fluids, e.g. blood, urine, saliva, etc. Many of such prior art optical analyzers are of such optical complexity size and weight, and have energy requirements such that they must be mounted stationary whereby the body fluid of interest must be taken from the source, such as a patient in a hospital bed or a patient coming to a doctor's office, whereafter the body fluid must be transmitted distantly to the location of the stationary optical analyzer for determination. While this procedure generally has worked well, it is known that the time lost between obtaining the fluid of interest, such as blood from a patient and transmitting the fluid distantly to the location of the stationary optical analyzer, may result in some deterioration of the fluid and thus some loss of quality in the subsequent analysis and analyte determination.

Accordingly, there exists a need in the art for an optical analyzer of sufficiently small size and weight whereby the optical analyzer is portable and may be, for example, taken to a patient in a hospital bed or purchased and taken home for home use.

## Summary of the Invention

An optical analyzer for determining an analyte in a fluid of interest such as a body fluid of interest satisfying the foregoing need and embodying the present invention may include a housing; combination optically transparent cuvette and lancet mounted removably in the housing, the cuvette may receive an optically transparent reagent test system for reacting with the body fluid to produce a change in at least one optical transmissive characteristic of the system indicative of the analyte; a cuvette carrier mounted slidably in the housing and for removably receiving the cuvette; a spring actuator mounted in the housing and connected to the cuvette carrier, the spring actuator may be compressed and released to advance the carrier and thereby advance the lancet into engagement with a portion of a body to produce the body fluid, and the spring actuator retract the lancet within the housing after the advancement; depth control apparatus for controlling the depth of penetration of the lancet into the body portion; an electro-optical system mounted in the housing in optical engagement with the cuvette and for passing a light beam through the cuvette and the reagent system and for receiving the light beam modified by the change in optical transmissive characteristic of the system and for transmitting computation signals indicative of the analyte to a computer; a computer mounted in the housing for receiving the computation signals and for comparing the computation signals against predetermined data to produce display signals indicative of the analyte; a display mounted in the housing and for receiving the display signals and for providing a visible display indicative of the analyte; and control switches mounted in the housing and connected to the computer for controlling the operation of the computer.

## Brief Description of the Drawings

FIG. 1 is a perspective view of an optical analyzer embodying the present invention;

FIG. 2 is a plan view, in partial cross-section and with the top portion of the housing removed, showing the optical analyzer embodying the present invention;

FIG. 3 is an irregular side view in elevation taken generally along the line 3-3 in FIG. 2;

FIG. 4 is a general block diagram of an electrical circuit which may be embodied in a computer included in the optical analyzer of the present invention;

FIG. 5 is a block diagram of a computer program which may be embodied in the computer; and

FIG. 6 is a flow chart of the test mode of the computer program.


Description of the Preferred Embodiment

Referring now to the FIGS., an optical analyzer embodying the present invention and indicated by general numerical designation 10 is illustrated. In the following description, the optical analyzer 10 will be described as embodied as an optical analyzer for determining the amount of glucose in human blood, but it will be expressly understood by those skilled in the art that the present invention is not so limited and has a wide variety of analyte determination applications. For example, and not by way of limitation, the optical analyzer of the present invention may be also used to determine cholesterol, blood urea nitrogen, etc.

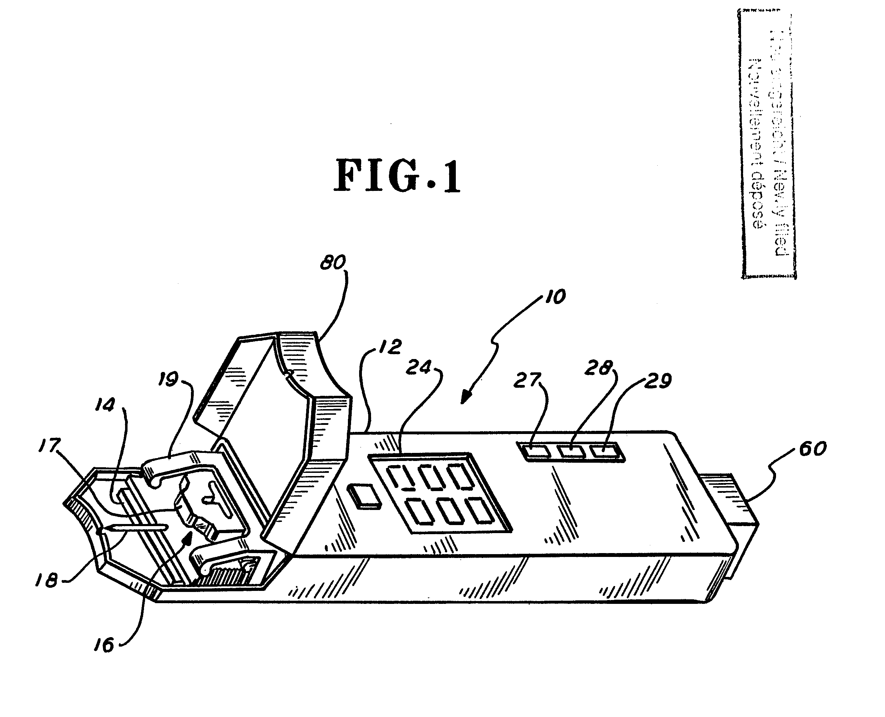

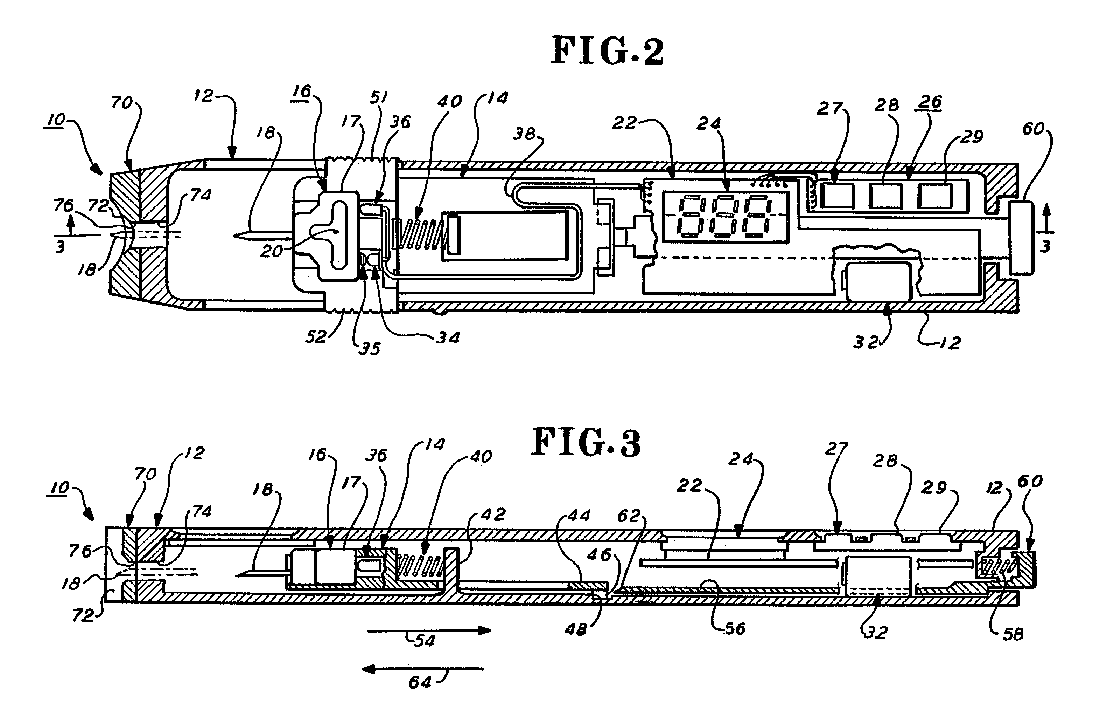

The optical analyzer 10 may include a housing 12 in which is slidably mounted a cuvette carrier 14 for removably receiving a disposable combination cuvette and lancet indicated by general numerical designation 16 with the cuvette being indicated by individual numerical designation 17 and with the lancet being indicated by individual numerical designation 18; the cuvette carrier 14 may include a clamp 19, FIG. 1, for removably clamping the cuvette 17 to the carrier. Combination cuvette and lancet 16 may be the IMPROVED CUVETTE disclosed in United States Patent Application Serial No. 888,752 filed in the United States on July 22, 1987, Peter M. Meserol et al. inventors, and assigned to the same assignee as the present invention. As illustrated in FIG. 2, the cuvette 17 is generally T-shaped in plan and is provided at the top with a T-shaped opening 20. As taught in the above-identified IMPROVED CUVETTE application, cuvette 17 may be filled with an optically transparent gel including a test reagent system for determining the amount of glucose present in human blood by a color change upon reaction of the blood, or a sample portion thereof, with the test reagent system which color change produces a change in the optical transmissive character of the gel and test reagent system contained within the cuvette 17; the optically transparent gel and test reagent system may be as disclosed in United States Patent Application Serial No. 888,755 filed in the United States on July 22, 1987, entitled GLUCOSE ASSAY, Rita C. Prodell et al. inventors, and assigned to the same assignee as the present invention. Accordingly, it will be assumed that the cuvette 17 has been filled with such optically transparent gel and test reagent system.

A computer 22 is mounted in the housing 12 and is connected to a display 24, for example by suitable surface mount technology in the manner known to those skilled in the art. Also connected to the computer 22, as shown, is a control panel indicated by general numerical designation 26 and including a calibrate button 27, a test button 28, and a memory button 29. A power supply, such as battery 32, is included to provide energy for operating the optical analyzer 10. A photoemitter 34, and collimating lens 35 if desired or required, and a photodetector 36, in optical engagement with the cuvette 17 are provided for passing a beam of light through the cuvette and the gel and test reagent system contained therein and for receiving the light beam modified by the above-noted change in optical transmissive characteristic of the gel and test reagent system and for transmitting computation signals over the flexible line 38 to the computer 22.

Spring 40 is mounted within the housing between the cuvette carrier 14 and the member 42 formed, by example, integrally with the housing 12 as illustrated in FIG. 3. As is further shown in FIG. 3, the rearward portion 44 of the cuvette carrier 14 is provided with a pair of downwardly extending, spaced apart detent members, only member 46 being shown in FIG. 3, for engaging the vertically upwardly extending portion 48 of the housing 12 upon the operator engaging the cuvette carrier 14, particularly the serrated portions 51 and 52 shown in FIG. 2, and forcing the cuvette carrier rearwardly in the direction of the arrow 54 of FIG. 3 to also compress the spring 40. A release lever 56 may be spring mounted in the housing 12, by spring 58 as shown, and may include a rearward release button 60 which upon being forced inwardly into the housing causes a camming portion 62 provided at the forward portion of the release 56 to engage the rearward portion 44 of the cuvette carrier 14 forcing the rearward portion upwardly to release the detent member 46 from the housing portion 48 whereupon the spring 40 advances the cuvette carrier and with it the combination cuvette and lancet 16 forwardly in the direction of the arrow 64 of FIG. 3 and to advance the lancet 16 into the forward positions shown in dashed outline in FIGS. 2 and 3 to puncture the finger of the user to a predetermined depth to produce a wound from which blood will flow. The depth of puncture may be controlled by the thickness of a tip 70 provided at the forward end of the housing and suitably secured thereto such as by a suitable adhesive. As shown in FIGS. 2 and 3, the tip 70 may be provided with an inwardly curved portion 72 to facilitate proper placement of the operator's finger at the forward portion of the optical analyzer 10 for puncture and production of blood. As shown in FIGS. 2 and 3, the tip 70 and the forward portion of the housing 12 are provided with coaxially aligned apertures 74 and 76 through which the lancet 18 may be advanced or extended.

It will be further understood by those skilled in the art that the spring 40 also retracts the cuvette carrier 14 and with it the cuvette 17 and lancet 18 to retract the lancet 18 within the housing 12 to prevent unwanted injury by the lancet. This retraction is accomplished by providing the slide carrier 14 with sufficient mass such that upon the spring 14 being compressed and the carrier released, the cuvette carrier will be forced forwardly sufficiently far to advance the lancet for finger puncture as described but also farther than the length of normal extension of the spring whereupon the spring will be placed in tension and will thereafter contract and retract the carrier slide and with it the cuvette and lancet as described.

The finger blood will be wiped across the T-shaped opening 20 of the cuvette 17 and will enter the cuvette and react with the test reagent system included in the gel contained therein, as described above, to cause a change in at least one optical transmissive characteristic of the gel and test reagent system, such as for example a change of color therein. The photoemitter 34 will pass a beam of light through the cuvette and the gel and test reagent system contained therein and the beam of light will be modified by the change in optical transmissive characteristic of the gel and test reagent system and the modified light beam will be received by the photodetector 36 which will transmit computation signals over the flexible line 38 to the computer 22. The computer will compare the computation signals against stored data to produce and transmit to the display 24 display signals indicative of the amount of glucose present in the blood, and the display will provide a digital display indicative thereof.

Referring again to FIG. 1, it will be noted that the upper portion of the housing 12 may be provided with a hinged or pivotally mounted portion 80 which may be pivoted upwardly, as shown in FIG. 1, to permit loading of the combination cuvette and lancet 16 into the optical analyzer 10 and which may thereafter be closed to operate the optical analyzer 10 as described above to puncture a finger to produce blood particularly a droplet of blood. Thereafter, the hinged portion 80 may again be pivoted upwardly as shown in FIG. 1 to permit the blood to be wiped across the T-shaped cuvette opening 20 to permit the blood to enter the cuvette and the plasma to diffuse into and react with the test reagent system included in the optical transparent gel, as described above.

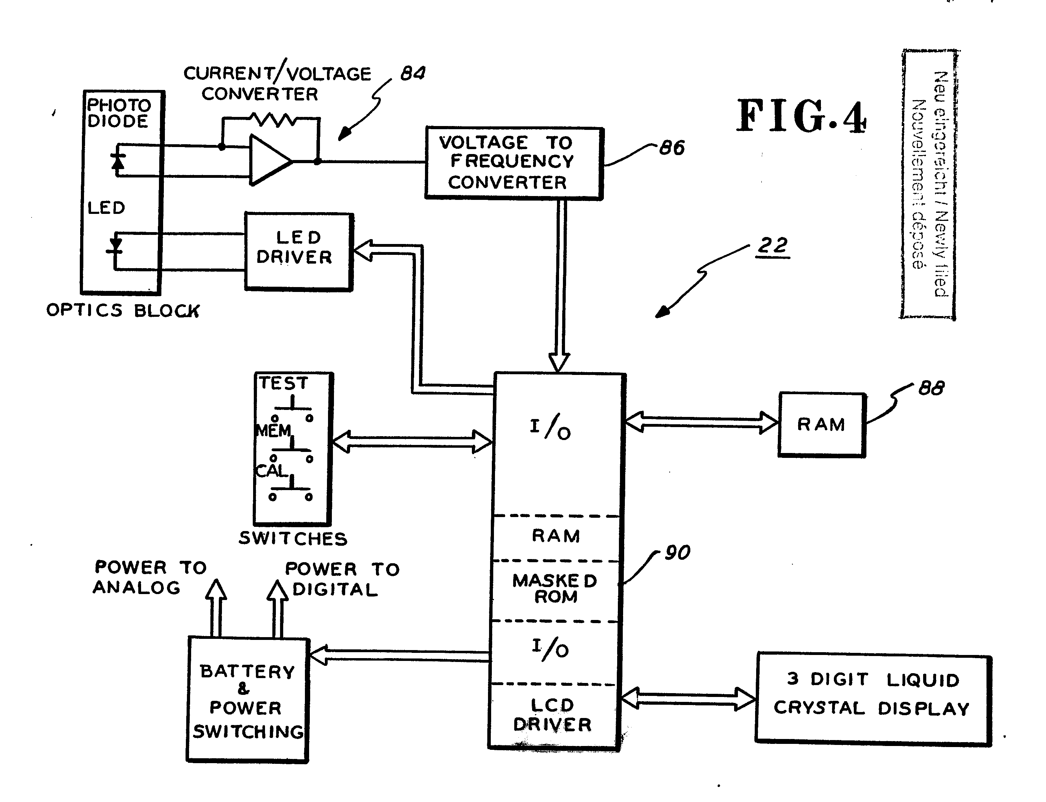

Referring now to FIG. 4, there is illustrated a block diagram of the computer 22 and, as noted generally above, it will be understood that the optical analyzer 10 of the present invention has three different modes of operation, namely test, calibrate and memory. It will be assumed that the operator, or patient, has produced a sample of blood and the blood has been wiped across the cuvette opening 20 and the plasma has entered and diffused into the gel and reacted with the test reagent system included therein, as described above. The operator then closes the pivotal member 80 (FIG. 1) and depresses the test key 28. The computer 22 then applies voltage to the circuitry and to the photoemitter 34 (FIG. 2) to generate and pass a beam of light into and through the cuvette 17 and the gel and test reagent system contained therein undergoing a change in at least one optical transmissive characteristic as noted above. The modified light beam is then received by the photodetector 36 (FIGS. 2 and 3) which generates a photocurrent proportional to the intensity of the light beam received. This current is amplified and converted to a voltage by the current-to-voltage converter 84 of FIG. 4; thereafter, the voltage is converted to a square wave at a frequency proportional to the applied voltage by the voltage-to-frequency converter 86. The computer 22 then determines the frequency of the square wave at fixed or predetermined intervals over the predetermined period of the test. Using this data, the computer 22 calculates the optical density change in the gel and test reagent system and determines the maximum rate of change of the test reagent system in the cuvette. Once this determination is made, the rate is equated to a blood glucose level stored in the computer memory and is thereafter displayed digitally by the display 24 and also stored in memory. Thereafter, the computer 22 automatically turns off and awaits the depression or closure of another switch.

Upon depression of the calibration key 27, the computer 22 functions in the same manner as the above-described test mode except that the sample placed in the cuvette 17 is not unknown blood from a patient but a sample of known glucose concentration to maintain the optical analyzer 10 in proper calibration.

Depression of the memory switch or button 29 permits the patient or user to display stored glucose readings; in one embodiment of the present invention one month's readings can be stored in the RAM 88 of FIG. 4.

As known to those skilled in the art, the computer 22 may consist of surface mounted components or may be incorporated into a suitable chip.

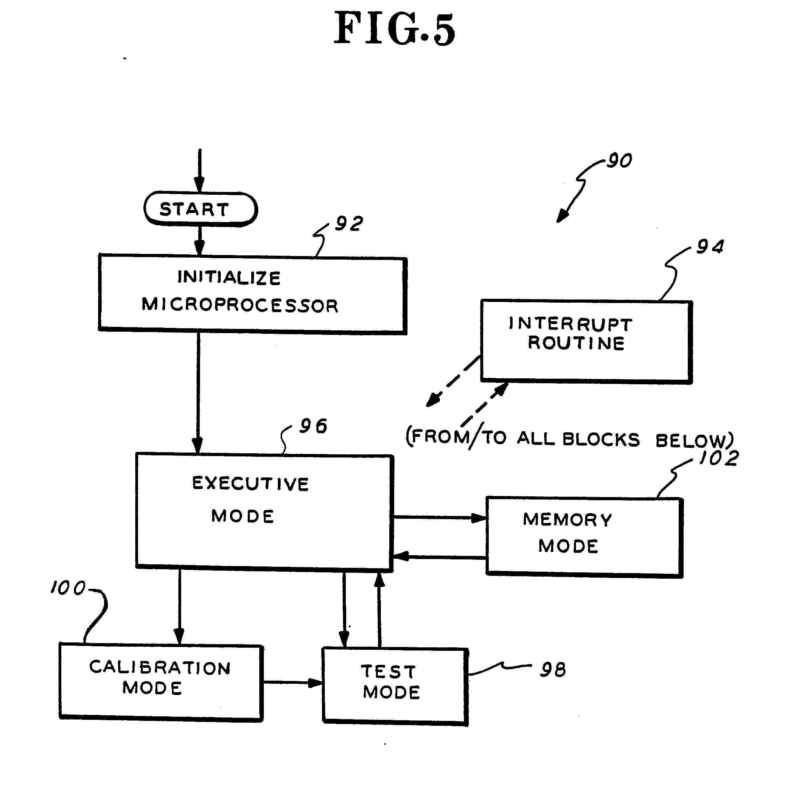

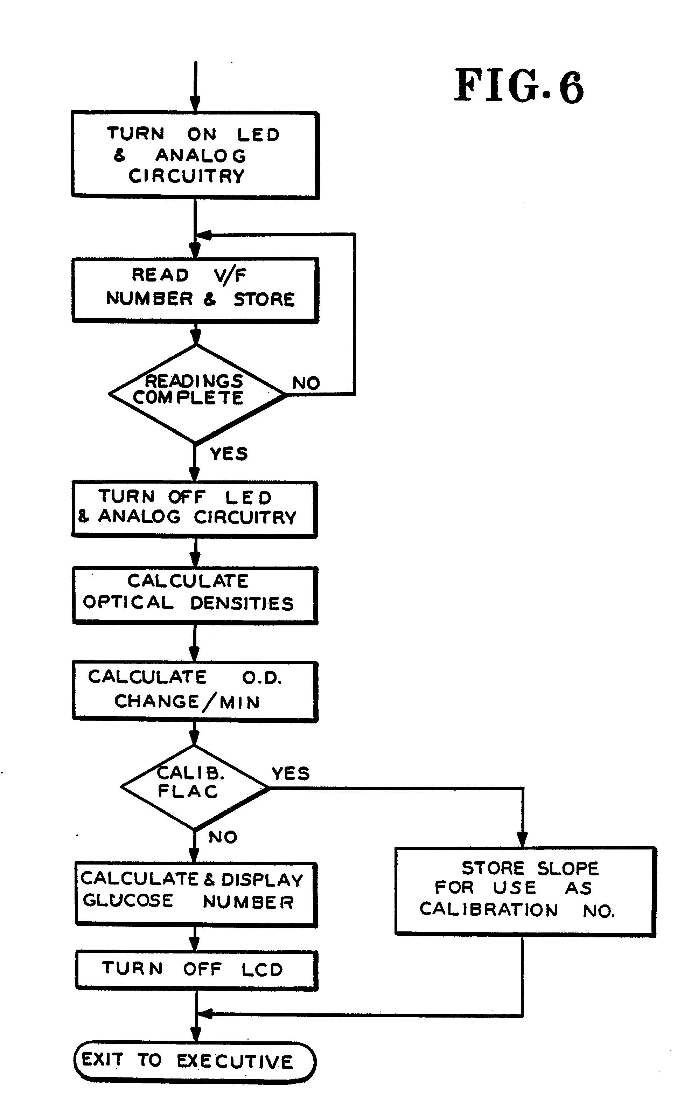

With regard to the computer software, it will be understood that the controlling element in the computer or microprocessor 22 (FIG. 4), in one embodiment of the present invention, was an eight bit CMOS masked ROM, e.g. ROM 90, and the stored computer program was configured into six basic blocks as illustrated in FIG. 5. Referring first to the initialization mode, block 92, this mode configures the computer control registers, sets timers and clock, clears the memory, and operates other test functions to allow the computer to perform the above-described test function. The interrupt mode, block 94, provides a routine which interrupts the main routine at a predetermined rate to perform the following functions: read the keys or switches 27, 28, and 29, start/stop the voltage to frequency converter 86 (FIG. 4), service the real time clock routine and decrement the test timers and counters. The executive mode, block 96, has the basic function of directing the computer program to the correct mode when one of the keys or buttons, 27, 28 or 29, is depressed. The test mode, block 98, is the primary function of the optical analyzer 10, and a computer or microprocessor 22, and illustrated in FIG. 6 is a flow chart of the test mode computer program; it will be understood by those skilled in the art that the flow chart of FIG. 6 will enable a computer programmer of ordinary skill to program the computer in any one of several different computer programming languages to cause the optical analyzer of the present invention to operate and perform analyte determination, such as the amount of glucose present in blood, as taught above. The calibration mode, block 100, is substantially the same as the test mode except that no blood glucose amount or level is displayed and, as noted above, the calibration mode is run with a known glucose sample to maintain the optical analyzer 10 in proper calibration. Lastly, the memory mode, block 102, allows the patient or operator of the optical analyzer 10 to have access to the memory, RAM 88 of FIG. 4, to display glucose levels or amounts previously stored; when a test is run, the glucose level or amount is stored in this memory as described above.

It will be understood by those skilled in the art that many variations and modifications may be made in the present invention without departing from the spirit and the scope thereof.

**Claims**

1. Optical analyzer (10) for determining an analyte in a body fluid of interest, comprising:
housing means (12);
a combination optically transparent cuvette and lancet (16) mounted removably in said housing means (12), said cuvette (17) for receiving an optically transparent reagent test system for reacting with said body fluid to produce a change in at least one optical transmissive characteristic of said system indicative of said analyte;
cuvette carrier means (14) mounted slidably in said housing means (12) and for receiving said cuvette (17);
spring means (40) mounted in said housing (12) and connected to said cuvette carrier means (14), said spring means (40) for being compressed and released to advance said carrier (14) and thereby advance said lancet (16) into engagement with a portion of a body to produce said body fluid, and said spring means (40) for retracting said lancet (16) within said housing (12) after said advancement;
depth control means (70, 72) for controlling the depth of penetration of said lancet (16) into said body portion;
electro-optical means (34, 36, 38) mounted in said housing (12) means in optical engagement with said cuvette (17) and for passing a light beam through said cuvette (17) and said reagent system and for receiving said light beam modified by said change in optical transmissive characteristic of said system and for producing computation signals from said received modified light beam indicative of said analyte and for transmitting said computation signals to computation means (22);
computation means (22) mounted in said housing means (12) for receiving said computation signals and for comparing said computation signals against predetermined data to produce display signals indicative of said analyte;
display means (24) mounted in said housing means (12) and for receiving said display signals and for providing a visible display indicative of said analyte; and
control means (26) mounted in said housing means (12) and connected to said computation means (22) for controlling the operation of said computation means (22).

2. Optical analyzer (10) according to Claim 1 wherein said control means (26) is also for operating said computation means (22) to recall and display previously stored analyte determination.

3. Optical analyzer according to Claim 1 wherein said reagent test system reacts with said body fluid at a rate of reaction, wherein said corresponding change in at least one optical transmissive characteristic has a rate of change, wherein said light beam is modified at a rate of modification, and wherein said computation means (22) determine the rate of change of said reaction from said received computation signals and compares the same against stored rates of change to determine said analyte and produce said display signals indicative thereof.

# FIG.1

0 254 203

# FIG.2

# FIG.3

0 254 203

FIG.4

0 254 203

# FIG.5

# FIG.6

TURN ON LED
& ANALOG
CIRCUITRY

READ V/F
NUMBER & STORE

READINGS
COMPLETE — NO

YES

TURN OFF LED
& ANALOG CIRCUITRY

CALCULATE
OPTICAL DENSITIES

CALCULATE O.D.
CHANGE/MIN

CALIB.
FLAC — YES

NO

CALCULATE & DISPLAY
GLUCOSE NUMBER

STORE SLOPE
FOR USE AS
CALIBRATION NO.

TURN OFF LCD

EXIT TO EXECUTIVE